# EUROPEAN PATENT APPLICATION

(11) **EP 4 062 904 A1**
(43) Date of publication of application: **28.09.2022**
(21) Application number: 20875299.8
(22) Date of filing: 04.09.2020
(51) Int. Cl.: A61K 9/127, A61K 47/10, A61K 47/12, A61K 47/14, A61K 9/48, A61K 31/202

(54) **LIQUID CRYSTALLINE STRUCTURE-FORMING OMEGA-3-FATTY ACID COMPOSITION**

(30) Priority: 11.10.2019 KR 20190125775
(71) Applicant: Imdpharm Inc., Yeongtong-gu Suwon-si, Gyeonggi-do 16226 (KR)
(72) Inventor: PARK, Young-Joon, Seoul 06583 (KR); JEON, Sangwon, Suwon-si Gyeonggi-do 16497 (KR); CHOI, Sook, Seoul 06583 (KR)
(74) Representative: Altmann Stößel Dick Patentanwälte PartG mbB
(86) International application number: PCT/KR2020/011962
(87) International publication number: WO 2021/071102

(57) **Abstract**

The present invention relates to a liquid crystalline structure-forming omega-3-fatty acid composition and, more specifically, provides an omega-3-fatty acid composition comprising: amphoteric lipids which form liquid crystalline structures when exposed to aqueous media such as gastrointestinal juice; omega-3 fatty acids or derivatives thereof; and a liquid crystalline structure-forming aid. The omega-3-fatty acid composition forms self-assembled lyotropic liquid crystals which are thermodynamically stable in structure, irrespective of digestion in the gastrointestinal tract, thereby promoting the solubilization and absorption rate of omega-3-fatty acids as well as providing preparations 30% or more smaller in size than conventional soft gel capsules of omega-3-fatty acid preparations. Thus, the composition can be taken with more ease by geriatric patients and the like and allows for provision of omega-3-fatty acid preparations increasing in *in-vivo* dissolution and absorption.

## Description

### [Technical Field]

The present invention relates to an omega-3-fatty acid composition including amphoteric lipids which form liquid crystalline structures when exposed to aqueous media such as gastrointestinal juice, and omega-3 fatty acids or derivatives thereof.

### [Background Art]

Omega-3-fatty acids are polyunsaturated fatty acids with a double bond at a third carbon atom from the end of a carbon chain, and are known to exist mainly in fish oil and also in plants such as sesame. Major omega-3-fatty acids involved in human physiology are α-linoleic acid, eicosapentaenoic acid (EPA), and docosahexaenoic acid (DHA), and are known to have physiological activities such as improving lipoprotein metabolism (improving blood lipid levels), suppressing and improving cardiovascular disease, improving hypertension, regulating the immune system, and acting on the development and functions of the eye/reproductive organs and nervous system.

In general, natural fish oil contains omega-3-fatty acids such as EPA and DHA in the form of triglycerides. The natural fish oil has been sold under various trade names such as Omacor, Lovaza, and Omtryg by processing, concentrating, and refining EPA and DHA in the form of ethyl esters, due to the problem of low purity.

In order for omega-3-fatty acids to pass through the biological membrane and be absorbed into the body, the omega-3-fatty acids are broken down into fatty acids by digestion under the action of bile acids and enzymes in the gastrointestinal tract, especially emulsification, and then need to be absorbed into the gastrointestinal epithelial cells, and again absorbed into the systemic circulatory system through re-esterification with glycerol groups and chylomicron formation in epithelial cells. At this time, there is a difference in absorption rate depending on the molecular structures of the omega-3-fatty acids.

In the case of omega-3-fatty acid ethyl esters, which are currently marketed as various products, due to the low activity of enzymes involved in digestion and the absence of a glycerol group, there is a problem in that the bioavailability is low compared to a triglyceride form.

Several preparations have been developed to improve the absorption rate of high-purity omega-3-fatty acids. For example, there are Epanova, an omega-3 concentrate in the form of free fatty acids, health functional foods in the form of re-esterification triglyceride and phospholipids combined, and the like, but there are still problems in dosage compliance of patients and the like due to a low absorption rate, a large difference in absorption rates before and after food intake (food effect), and a large size of a soft capsule preparation.

To improve the bioavailability of omega-3-fatty acids, in WO 2012/032417, there is disclosed a lipid concentrate capable of forming a self-nanoemulsifying drug delivery system (SNEDDS), a self-microemulsifying drug delivery system (SMEDDS), or self-emulsifying drug delivery systems (SEDDS) with omega-3-fatty acid ethyl ester and triglyceride in an aqueous solution including at least one free fatty acid, and at least one surfactant. However, in the case of the self- emulsifying system, in order to be emulsified in the aqueous solution, a surfactant and a co-surfactant in a composition ratio several times larger than that of an oil component need to be contained, and if the ratio of the surfactant is low, the self-emulsifying emulsion is not formed well, and the omega-3-fatty acids, as the oil component, are not emulsified in the gastrointestinal tract, but separated, and thus are not absorbed properly. In addition, even in order to form a general emulsion system rather than the self-emulsifying system, a stronger shear force than *in-vivo* conditions such as peristalsis of the gastrointestinal tract is required, which may cause insufficient effects in a body environment. In addition, the emulsion is thermodynamically unstable, and a large amount of surfactant and co-surfactant is required for forming the emulsion system. Therefore, in order to form such a self-emulsifying system, since the total weight of the preparation increases due to the oil component and a large amount of surfactant for emulsifying the oil component, and the size of the preparation increases, there is a disadvantage that it is difficult for a patient to take a drug.

For effective drug delivery of the omega-3-fatty acids, there is disclosed a liquid crystal composition including eicosapentaenoic acid; and glyceryl monooleate and prepared after dissolving the eicosapentaenoic acid and glyceryl monooleate in an organic solvent. However, instead of a system in which liquid crystals are spontaneously formed when exposed to an aqueous solution, liquid crystal particles may be formed through strong external energy (ultrasonication) after mixing under a condition of an organic solvent (chloroform), volatilizing the organic solvent and adding a limited environment (pH 7.0 phosphate buffer). It is a preparation applicable to actual clinical practice, but there are limitations in application.

Therefore, there is a need for development of an omega-3-fatty acid composition with an increased ease of administration by minimizing the absorption effect of food intake and improving the absorption rate in the gastrointestinal tract to reduce the size of the preparation.

### [Disclosure]

### [Technical Problem]

In order to solve the problems, an object of the present invention is to provide a composition including amphoteric lipids which form liquid crystalline structures when exposed to aqueous media such as gastrointestinal juice, a self-liquid crystalline structure-forming aid, and omega-3-fatty acids or derivatives thereof to improve the absorption rate of the omega-3-fatty acids, thereby providing omega-3-fatty acid preparations with reduced sizes, and to provide an omega-3-fatty acid composition with improved administration.

### [Technical Solution]

One aspect of the present invention provides an omega-3-fatty acid composition including a liquid crystal former comprising amphoteric lipids containing a nonpolar tail group having 14 to 20 carbon atoms (C₁₄ to C₂₀) and a polar head group having a hydroxyl (-OH) or carboxyl group (-COOH); omega-3-fatty acids or derivatives thereof; and liquid crystalline structure-forming aids of lipid components, wherein the omega-3-fatty acid composition spontaneously forms liquid crystals in an aqueous medium.The omega-3-fatty acid composition of the present invention may include the liquid crystal former (including the liquid crystalline structure-forming aid) comprising the amphoteric lipids containing the nonpolar tail group having 14 to 20 carbon atoms (C₁₄ to C₂₀) and the polar head group having the hydroxyl (-OH) or carboxyl group (-COOH) and the omega-3-fatty acids or derivatives thereof in the range of 20 to 60 parts by weight and 40 to 80 parts by weight, based on 100 parts by weight of the composition, respectively. A weight ratio of the liquid crystal former and the liquid crystalline structure-forming aid may be 10 : 1 to 2 : 8.

Another aspect of the present invention provides an omega-3-fatty acid oral preparation including the omega-3-fatty acid composition and a pharmaceutically acceptable carrier.

Yet another aspect of the present invention provides an omega-3-fatty acid gelatin soft capsule including the omega-3-fatty acid composition.

### [Advantageous Effects]

According to the present invention, the liquid crystal former comprising amphoteric lipids spontaneously forms lyotropic liquid crystals with a thermodynamically stable structure regardless of digestion in the gastrointestinal tract to solubilize omega-3-fatty acids, thereby significantly improving dissolution, so that it is expected to promote *in-vivo* absorption rate. Accordingly, compared to conventional preparations, which have typically contained 1000 mg by weight of omega-3-fatty acids, the *in-vivo* absorption rate is improved to obtain the same effect even if a lower dose of omega-3-fatty acids is administered than the conventional preparations. Thus, since there may be provided a capsule size in which the amount of main components contained is small and the size of the preparation is reduced, it is possible to provide omega-3-fatty acid preparations taken with more ease by geriatric patients and the like and increased in *in-vivo* dissolution and absorption.

### [Description of Drawings]

FIG. 1 illustrates dissolution test results of *in-vitro* EPA ethyl esters of compositions of Examples 6, 8, and 68 and Comparative Examples 1 and 2.
FIG. 2 illustrates dissolution test results of *in-vitro* DHA ethyl esters of compositions of Examples 6, 8, and 68 and Comparative Examples 1 and 2.
FIG. 3 illustrates a result of measuring particle sizes of liquid crystals when the compositions of Examples 6, 8, and 68 are dispersed in an *in-vitro* condition of *in-vivo* gastrointestinal tract.
FIG. 4 illustrates a result of confirming isotropic as a polarization microscope analysis result of liquid crystals having a cubic structure formed when the compositions of Examples 6, 8, and 68 are dispersed in an *in-vitro* condition of *in-vivo* gastrointestinal tract.

### [Best Mode of the Invention]

Hereinafter, the present invention will be described in detail.

The present inventors have conducted various studies to develop a composition containing omega-3-fatty acids with improved dosage compliance of patients by increasing the absorption rate, reducing a dosage, and reducing the size of the preparation, found that a liquid crystal system including amphoteric lipids capable of spontaneously forming (self-assembly) lyotropic liquid crystal structures in the gastrointestinal tract was formulated with omega-3-fatty acids or derivatives thereof to have higher absorption rate than conventional preparations of omega-3-fatty acids, and then completed the present invention.

Therefore, the present invention provides an omega-3-fatty acid composition including a liquid crystal former comprising two or more amphoteric lipids containing a nonpolar tail group having 14 to 20 carbon atoms (C₁₄ to C₂₀) and a polar head group having a hydroxyl (-OH) or carboxyl group (-COOH); omega-3-fatty acids or derivatives thereof; and a liquid crystalline structure-forming aid of a lipid component, wherein the omega-3-fatty acid composition forms liquid crystals in an aqueous medium.

The omega-3-fatty acid composition may include, based on 100 parts by weight of the composition, 20 to 60 parts by weight of the liquid crystal former, and 40 to 80 parts by weight of the omega-3-fatty acids or derivatives thereof.

In addition, the omega-3-fatty acid composition may have a weight ratio of the liquid crystal former and the liquid crystalline structure-forming aid of 10 : 1 to 2 : 8.

More specifically, the omega-3 fatty acids may include omega-3, omega-3-fatty acids, omega-3 derivatives (ethyl ester derivatives or carboxylic acid derivatives, omega-3 complexes), or DHA, eicosapentaenoic acid (EPA), etc., which are omega-3 internal components, and these omega-3 fatty acids may be included in an amount of 40 parts by weight to 80 parts by weight based on 100 parts by weight of the total composition. When the omega-3 fatty acids are included in an amount of 40 parts by weight or less, the size of the preparation increases or the amount of capsules taken per dose increases, and as a result, it may be difficult for the patient to take. When the omega-3 fatty acids are included in an amount of 80 parts by weight or more, the ratio of the liquid crystal former is insufficient, and thus the liquid crystalline structures in the living body are not well formed, so that the *in-vivo* dissolution of the omega-3-fatty acids may be lowered and *in-vivo* absorption may not be improved.

The liquid crystal former is preferably 20 to 60 parts by weight based on 100 parts by weight of the total composition. When the liquid crystal former is contained in an amount of less than 20 parts by weight, it is not easy to form a liquid crystal structure in the *in-vivo* condition, and thus, the *in-vivo* dissolution of omega-3-fatty acids is reduced and thus *in-vivo* absorption may not be improved. In addition, when the liquid crystal former is contained in an amount of more than 60 parts by weight, the content of the main component, omega-3-fatty acids is lowered, so that the size of the preparation increases and the amount of capsules taken per dose increases, making it difficult for patients to take medication.

The liquid crystal former of the present invention has a lipid solution form, and means amphoteric lipids spontaneously forming liquid crystal structure particles of 1000 nm or less, preferably 500 nm or less when exposed to an excess of aqueous media including water, gastrointestinal juice, and the like, and a material including the same. The amphoteric lipids include a polar head group and one or two nonpolar tail groups in a molecular structure, and serve to form nanometer-scale liquid crystals in aqueous media to increase the solubility and dissolution of omega-3-fatty acids in an *in-vivo* condition, thereby playing an important role in increasing *in-vivo* absorption. As the liquid crystal structures formed in such an environment, a reversed hexagonal phase, a reversed cubic phase, a multi-lamellar vesicle, etc. may be generated, and the properties of the liquid crystalline structures are not limited, and the structures have an advantage of forming a polar region and a nonpolar region to solubilize both a polar material and a nonpolar material.

More specifically, the amphoteric lipids are amphoteric lipids comprising 1 to 2 nonpolar tail groups having 14 to 20 carbon atoms and at least one polar head group having a hydroxy or carboxyl group, and more preferably, may be one or a combination of two or more selected from the group consisting of neutral acyl/diacyl glycerol, phytantriol, phospholipids, etc, but are not limited thereto.

The neutral acyl/diacyl glycerol may be one or a combination of two or more selected from the group consisting of glyceryl monooleate, glyceryl monolinoleate, glyceryl palmitate, glyceryl dioleate, glyceryl dipalmitate, glyceryl phytanoate, glyceryl palmitoleate, glyceryl distearate, glyceryl dielaidiate, and glyceryl dilinoleate.

The liquid crystalline structure-forming aid is a lipid component that helps the liquid crystal former to self-form liquid crystalline structures in an *in-vivo* condition (e.g., in the gastrointestinal tract). The liquid crystalline structure-forming aid includes materials with good compatibility with omega-3-fatty acids and excellent biocompatibility, and for example, the liquid crystalline structure-forming aid may be one or a combination of two or more selected from the group consisting of phospholipids, unsaturated fatty acids, tocopherol acetate, cholesterols, and vegetable oils, but is not limited thereto.

The phospholipids include a polar head group and two nonpolar tail groups, and may include various derived or synthetic phospholipids, including phospholipids derived from soybean or egg yolk. Preferably, the phospholipids may be one or a combination of two or more selected from the group consisting of phosphatidylcholine, phosphatidylethanolamine, phosphatidylserine, phosphatidylglycerine, phosphatidylinositol, phosphatidic acid, and sphingomyelin, but is not limited thereto.

The unsaturated fatty acids are liquid unsaturated fatty acids at room temperature and a bio-derived component having 1 to 3 double bonds and widely present in animals and plants, have excellent biocompatibility, and serve to form lyotropic liquid crystal structures such as a reversed hexagonal phase, a reversed cubic phase, and a multi-lamellar vesicle in the gastrointestinal tract with the amphoteric lipids forming the liquid crystals. Accordingly, it has been confirmed through the present invention that the solubilization and absorption promoting effects of omega-3-fatty acids were increased.

The liquid unsaturated fatty acids may be one or a combination of two or more selected from the group consisting of oleic acid, linoleic acid, myristoleic acid, palmitoleic acid, and 11-eicosenoic acid, but are not limited thereto.

The omega-3-fatty acids may be selected from eicosapentaenoic acid (EPA) or docosahexaenoic acid (DHA), and the derivatives thereof may be selected from the group consisting of ethyl esters, triglycerides, free fatty acids, and phospholipids of omega-3-fatty acids, but are not limited thereto.

The composition may further include one or more antioxidants selected from the group consisting of α-tocopherol acetate, butylhydroxyanisole (BHA), and butylhydroxytoluene (BHT).

The composition may spontaneously form lyotropic liquid crystal structures in the gastrointestinal tract.

In addition, the present invention provides an omega-3-fatty acid oral preparation including the omega-3-fatty acid composition and a pharmaceutically acceptable carrier.

In addition, the present invention provides an omega-3-fatty acid soft capsule including the omega-3-fatty acid composition and a pharmaceutically acceptable carrier.

The soft capsule may be filled with 150 to 1500 mg of the omega-3-fatty acid composition, more preferably 600 to 800 mg of the omega-3-fatty acid composition, but is not limited thereto.

### [Modes for the Invention]

Hereinafter, the present invention will be described in detail with reference to Examples for understanding. However, the following Examples are merely illustrative of the contents of the present invention, and the scope of the present invention is not limited to the following Examples. Examples of the present invention will be provided for more completely explaining the present invention to those skilled in the art.

### <Examples 1 to 20> Preparation of compositions including omega-3-fatty acid ethyl esters

Examples 1 to 20 containing omega-3-fatty acid ethyl esters (Omega-3>90EE, K.D.Pharma) were prepared according to components and amounts in Table 1 below. Specifically, in a glass vial, omega-3-fatty acid ethyl ester, liquid crystal formers (glyceryl monooleate, glyceryl dioleate, and phytantriol), and liquid crystalline structure-forming aids (phosphatidylcholine, oleic acid, tocopherol acetate, and cholesterol) were added, and then stirred and mixed at room temperature with a magnetic stirrer. The total medium scale was prepared at 20 g per preparation.

**[Table 1]**

| (Unit: mg) | Examples | | | | | | | | | | | | | | | | | | | |
|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 |
| omega-3-fatty acid ethyl ester | 32 0 | 32 0 | 32 0 | 60 0 | 60 0 | 50 0 | 50 0 | 50 0 | 50 0 | 50 0 | 32 0 | 50 0 | 50 0 | 50 0 | 60 0 | 32 0 | 50 0 | 50 0 | 50 0 | 60 0 |
| Glyceryl monooleate | 39 0 | 12 9 | 21 5 | 11 0 | 75 | 20 0 | 12 5 | 24 0 | 12 5 | 60 | | | | | | | | | | |
| Glyceryl dioleate | | | | | | | | | | | 35 5 | 12 5 | 17 0 | 12 5 | 11 0 | | | | | |
| Phytantriol | | | | | | | | | | | | | | | | 35 5 | 12 5 | 17 0 | 12 5 | 11 0 |
| Phosphatid ylcholine | 40 | 30 1 | 21 5 | 40 | 75 | | | | | | 75 | 12 5 | | | | 75 | 12 5 | | | |
| Oleic acid | | | | | | 50 | 12 5 | | 11 5 | 24 0 | | | 80 | 12 5 | 40 | | | 80 | 12 5 | 40 |
| Tocopherol acetate | | | | | | | | 10 | 10 | | | | | | | | | | | |
| Cholesterol | | | | | | | | | | 10 | | | | | | | | | | |

### <Examples 21 to 40> Preparation of compositions including omega-3-fatty acid triglycerides

Examples 21 to 40 containing omega-3-fatty acid triglycerides (KD 480320 TG, K.D.Pharma) were prepared according to components and amounts in Table 2 below. Specifically, in a glass vial, omega-3-fatty acid triglyceride, liquid crystal formers (glyceryl monooleate, glyceryl dioleate, and phytantriol), and liquid crystalline structure-forming aids (phosphatidylcholine, oleic acid, tocopherol acetate, and cholesterol) were added, and then stirred and mixed at room temperature with a magnetic stirrer. The total medium scale was prepared at 20 g per preparation.

**[Table 2]**

| (Unit: mg) | Examples | | | | | | | | | | | | | | | | | | | |
|---|---|---|---|---|---|---|---|---|---|---|---|---|---|---|---|---|---|---|---|---|
| | 21 | 22 | 23 | 24 | 25 | 26 | 27 | 28 | 29 | 30 | 31 | 32 | 33 | 34 | 35 | 36 | 37 | 38 | 39 | 40 |
| Omega-3-fatty acid triglyceride | 32 0 | 32 0 | 32 0 | 60 0 | 60 0 | 50 0 | 50 0 | 500 | 50 0 | 50 0 | 32 0 | 50 0 | 50 0 | 50 0 | 60 0 | 32 0 | 50 0 | 50 0 | 50 0 | 60 0 |
| Glyceryl monooleate | 39 0 | 35 5 | 21 5 | 11 0 | 75 | 20 0 | 12 5 | 240 | 12 5 | 12 5 | | | | | | | | | | |
| Glyceryl dioleate | | | | | | | | | | | 35 5 | 12 5 | 17 0 | 12 5 | 11 0 | | | | | |
| Phytantriol | | | | | | | | | | | | | | | | 35 5 | 12 5 | 17 0 | 12 5 | 11 0 |
| Phosphatidyl choline | 40 | 75 | 21 5 | 40 | 75 | | | | | | 75 | 12 5 | | | | 75 | 12 5 | | | |
| Oleic acid | | | | | | 50 | 12 5 | | 11 5 | 11 5 | | | 80 | 12 5 | 40 | | | 80 | 12 5 | 40 |
| Tocopherol acetate | | | | | | | | 10 | 10 | | | | | | | | | | | |
| Cholesterol | | | | | | | | | | 10 | | | | | | | | | | |

### <Examples 41 to 60> Preparation of compositions including omega-3-fatty acid phospholipids

Examples 41 to 60 containing omega-3-fatty acid phospholipids (Omega3MPL, CLS technology) were prepared according to components and amounts in Table 3 below. Specifically, in a glass vial, omega-3-fatty acid phospholipids, liquid crystal formers (glyceryl monooleate, glyceryl dioleate, and phytantriol), and liquid crystalline structure-forming aids (phosphatidylcholine, oleic acid, tocopherol acetate, and cholesterol) were added, and then stirred and mixed at room temperature with a magnetic stirrer. The total medium scale was prepared at 20 g per preparation.

**[Table 3]**

| (Unit: mg) | Examples | | | | | | | | | | | | | | | | | | | |
|---|---|---|---|---|---|---|---|---|---|---|---|---|---|---|---|---|---|---|---|---|
| | 41 | 42 | 43 | 44 | 45 | 46 | 47 | 48 | 49 | 50 | 51 | 52 | 53 | 54 | 55 | 56 | 57 | 58 | 59 | 60 |
| Omega-3-fatty acid phospholipids | 32 0 | 32 0 | 32 0 | 60 0 | 60 0 | 50 0 | 50 0 | 50 0 | 50 0 | 50 0 | 32 0 | 50 0 | 50 0 | 50 0 | 60 0 | 32 0 | 50 0 | 50 0 | 50 0 | 60 0 |
| Glyceryl monooleate | 39 0 | 35 5 | 21 5 | 11 0 | 75 | 20 0 | 12 5 | 24 0 | 12 5 | 12 5 | | | | | | | | | | |
| Glyceryl dioleate | | | | | | | | | | | 35 5 | 12 5 | 17 0 | 12 5 | 11 0 | | | | | |
| Phytantriol | | | | | | | | | | | | | | | | 35 5 | 12 5 | 17 0 | 12 5 | 11 0 |
| Phosphatidylc holine | 40 | 75 | 21 5 | 40 | 75 | | | | | | 75 | 12 5 | | | | 75 | 12 5 | | | |
| Oleic acid | | | | | | 50 | 12 5 | | 11 5 | 11 5 | | | 80 | 12 5 | 40 | | | 80 | 12 5 | 40 |
| Tocopherol acetate | | | | | | | | 10 | 10 | | | | | | | | | | | |
| Cholesterol | | | | | | | | | | 10 | | | | | | | | | | |

### <Examples 61 to 70> Preparation of compositions including omega-3-fatty acid ethyl esters and antioxidants

Examples 61 to 70 containing omega-3-fatty acid ethyl esters (Omega-3>90EE, K.D.Pharma) were prepared according to components and amounts in Table 4 below. Specifically, in a glass vial, omega-3-fatty acid ethyl ester, a liquid crystal former (glyceryl monooleate), liquid crystalline structure-forming aids (phosphatidylcholine, and oleic acid), and antioxidants (tocopherol acetate, butylhydroxyanisole (BHA), butylhydroxytoluene (BHT)) were added, and then stirred and mixed at room temperature with a magnetic stirrer. The total medium scale was prepared at 20 g per preparation.

**[Table 4]**

| (Unit: mg) | Examples | | | | | | | | | |
|---|---|---|---|---|---|---|---|---|---|---|
| | 61 | 62 | 63 | 64 | 65 | 66 | 67 | 68 | 69 | 70 |
| Omega-3-fatty acid ethyl ester | 500 | 500 | 500 | 500 | 500 | 500 | 500 | 500 | 500 | 500 |
| Glyceryl monooleate | 163 | 163 | 163 | 163 | 110 | 110 | 110 | 125 | 125 | 125 |
| Phosphatidylcholine | 82 | | | | 90 | 90 | 90 | | | |
| Oleic acid | | 82 | 82 | 82 | 45 | 45 | 45 | 120 | 120 | 120 |
| Tocopherol acetate | 5 | 5 | | | 5 | | | 5 | | |
| Butylhydroxyanisole (BHA) | | | 5 | | | 5 | | | 5 | |
| Butylhydroxytoluene (BHT) | | | | 5 | | | 5 | | | 5 |

### <Comparative Examples 1 and 2> Omega-3-fatty acid ethyl ester and commercial preparation (Omacor^{®} Soft Capsule)

In the present invention, Omacor^{®} soft capsule and omega-3-fatty acid ethyl ester [Omega-3>90EE, K.D.Pharma] were used as Comparative Examples 1 and 2, respectively.

### <Experimental Example 1> In vitro dissolution tests of compositions including omega-3-fatty acids ethyl esters

In order to predict the absorption behavior in the gastrointestinal tract, *in vitro* dissolution tests for Examples 6, 8, and 68 and Comparative Examples 1 and 2 were performed.

Specifically, the composition prepared in Example was filled in a hard capsule (No. 00) so as to be 500 mg as omega-3-fatty acids ethyl ester. A test solution (fed state simulated intestinal fluid, FeSSIF) of pH 5.0 containing 15.000 g of Triton X-100, 8.250 g of sodium taurocholic acid, 2.950 g of soybean-derived phosphatidylcholine, 8.650 g of acetic acid, 11.874 g of sodium chloride, and 4.040 g of sodium hydroxide per 1 L of the test solution was prepared, and then dissolution tests and analysis were performed under the following conditions.

The dissolution tests were carried out in an apparatus (paddle method) of a dissolution test method 2 among general test methods of the Korean Pharmacopoeia. A FeSSIf effluent of pH 5.0 containing 900 mL of 1.5% (w/v) Triton X-100 was maintained at 37.0 ± 0.5°C, and the rotation speed of a paddle was set to 100 rpm. 5 mL of a sample was collected, and the dissolution solution was not separately supplemented after collection. The collected sample was analyzed in a high performance liquid chromatography (HPLC) apparatus, and the analysis conditions were as follows.

### 1. HPLC analysis conditions

- Column: Column (Aegispak C18-L) filled with octadecyl silylated silica gel for liquid chromatography with a particle diameter of 5 µm in stainless steel tube having a length of 150 mm and an inner diameter of 4.6 mm
- Moving phase: Purified water: Acetonitrile = 10 : 90 (v/v)
- Wavelength: 215 nm (ultraviolet absorbance spectrophotometer)
- Flow rate: 1.0 ml/min
- Column temperature: 25°C
- Injection amount: 10 µl

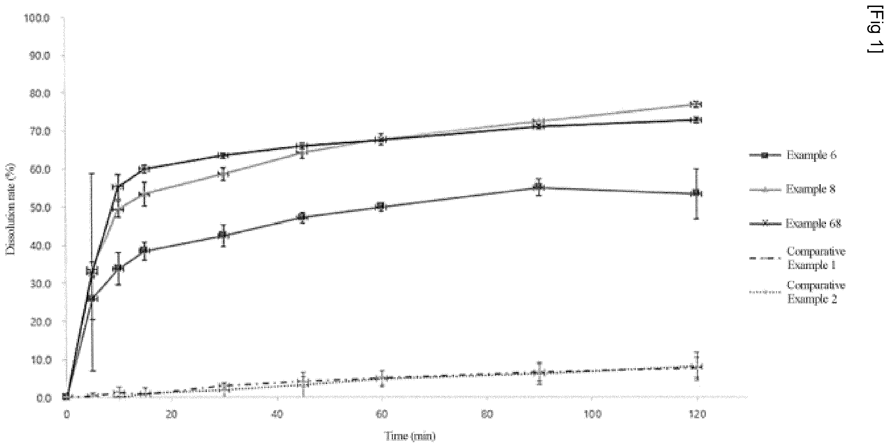

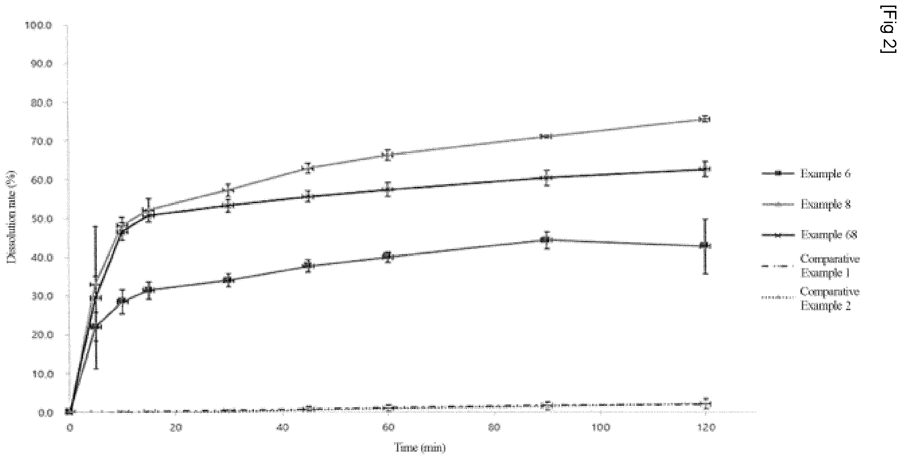

As a result, as illustrated in FIGS. 1 and 2, it was confirmed that the compositions of Examples 6, 8, and 68 according to the present invention promoted the absorption rate of omega-3-fatty acids in the artificial intestinal fluid as compared with Comparative Examples 1 and 2 which were conventional omega-3-fatty acid preparations, and accordingly, it can be expected that the *in-vivo* absorption rate will increase.

### <Experimental Example 2> Confirmation of liquid crystal structure particles containing omega-3-fatty acids

In order to evaluate whether to spontaneously form lyotropic liquid crystal structures in the gastrointestinal tract, polarized light microscopy and dynamic light scattering particle size analysis for Examples 6, 8, and 68 were used.

Specifically, a test solution (fed state simulated intestinal fluid, FaSSIF) of pH 5.0 containing 8.250 g of sodium taurocholic acid, 2.950 g of soybean-derived phosphatidylcholine, 8.650 g of acetic acid, 11.874 g of sodium chloride, and 4.040 g of sodium hydroxide per 1 L of purified water, and a test solution (pre-fed state simulated intestinal fluid, FaSSIF) of pH 6.5 containing 1.700 g of sodium taurocholic acid, 0.600 g of soybean-derived phosphatidylcholine, 3.400 g of sodium dihydrogen phosphate, 6.200 g of sodium chloride, and 0.400 g of sodium hydroxide per 1 L of purified water were prepared. The composition prepared in Example was filled into soft capsules so as to be 500 mg as omega-3-fatty acid ethyl ester, and exposed to 900 ml of the simulated intestinal fluid before and after meals under the dissolution test conditions of Experimental Example 1 for 30 minutes, and then polarization microscope and dynamic light scattering particle size analysis were performed under the following conditions.

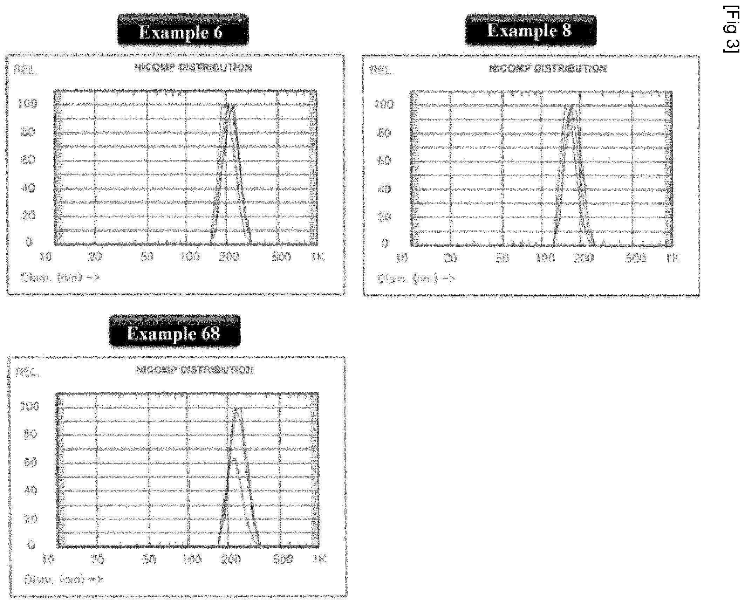

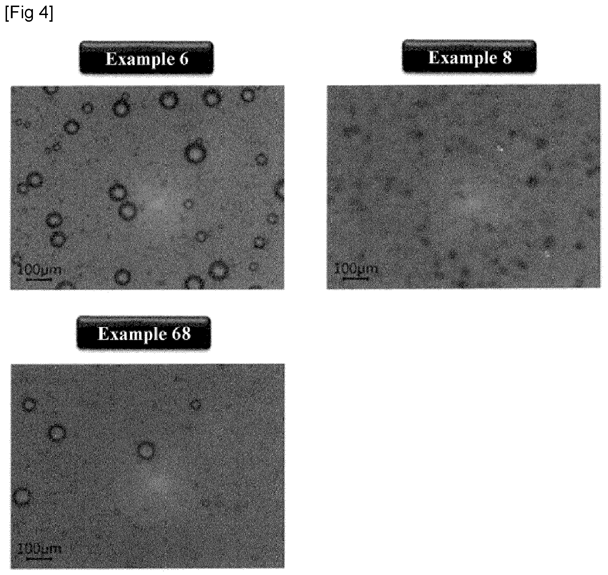

As a result, as illustrated in FIGS. 3 and 4, it can be confirmed that the composition of Example according to the present invention forms liquid crystal particles in the simulated intestinal fluid.

### <Experimental Example 3> Preparation of soft capsules of composition containing omega-3-fatty acids

To prepare soft capsules filled with a composition containing omega-3-fatty acids, soft capsules with various sizes (5 oval, 12 oblong, 14 oval, etc.) were produced after preparing a gelatin film.

Specifically, a gelatin film containing 415 g of succinic acid gelatin, 130 g of concentrated glycerin, 305 g of purified water, and 150 g of amorphous sorbitol solution per 1 kg of the film was prepared and after 1 day of aging, filled with a composition containing omega-3-fatty acids at a film thickness of 0.080 mm, and then dried for about 2 days under 25°C/blowing conditions, and the soft capsule was produced.

As described above, specific parts of the present invention have been described in detail, and it will be apparent to those skilled in the art that these specific techniques are merely preferred embodiments, and the scope of the present invention is not limited thereto. Therefore, the substantial scope of the present invention will be defined by the appended claims and their equivalents.

## Claims

1. An omega-3-fatty acid composition comprising:
a liquid crystal former comprising two or more amphoteric lipids containing a nonpolar tail group having 14 to 20 carbon atoms (C₁₄ to C₂₀) and a polar head group having a hydroxyl (-OH) or carboxyl group (-COOH);
omega-3-fatty acids or derivatives thereof; and
a liquid crystalline structure-forming aid of a lipid component, wherein the omega-3-fatty acid composition forms liquid crystals in an aqueous medium.

2. The omega-3-fatty acid composition of claim 1, wherein the omega-3-fatty acid composition includes, based on 100 parts by weight of the composition, 20 to 60 parts by weight of the liquid crystal former, and 40 to 80 parts by weight of the omega-3-fatty acids or derivatives thereof.

3. The omega-3-fatty acid composition of claim 2, wherein the omega-3-fatty acid composition has a weight ratio of the liquid crystal former and the liquid crystalline structure-forming aid of 10 : 1 to 2 : 8.

4. The omega-3-fatty acid composition of claim 1, wherein the amphoteric lipids are one or a combination of two or more selected from the group consisting of neutral acyl/diacyl glycerol, phytantriol, and phospholipids.

5. The omega-3-fatty acid composition of claim 4, wherein the neutral acyl/diacyl glycerol is one or a combination of two or more selected from the group consisting of glyceryl monooleate, glyceryl monolinoleate, glyceryl palmitate, glyceryl dioleate, glyceryl dipalmitate, glyceryl phytanoate, glyceryl palmitoleate, glyceryl distearate, glyceryl dielaidiate, and glyceryl dilinoleate.

6. The omega-3-fatty acid composition of claim 1, wherein the liquid crystalline structure-forming aid is one or a combination of two or more selected from the group consisting of phospholipids, unsaturated fatty acids, tocopherol acetate, cholesterols, and vegetable oils.

7. The omega-3-fatty acid composition of claim 6, wherein the phospholipids are one or a combination of two or more selected from the group consisting of phosphatidylcholine, phosphatidylethanolamine, phosphatidylserine, phosphatidylglycerine, dipalmitoyl phosphatidylcholine, distearyl phosphatidylcholine, phosphatidylinositol, phosphatidic acid, and sphingomyelin.

8. The omega-3-fatty acid composition of claim 6, wherein the unsaturated fatty acids are one or a combination of two or more selected from the group consisting of oleic acid, linoleic acid, myristoleic acid, palmitoleic acid, and 11-eicosenoic acid.

9. The omega-3-fatty acid composition of claim 1, wherein the omega-3-fatty acids are selected from eicosapentaenoic acid (EPA) or docosahexaenoic acid (DHA).

10. The omega-3-fatty acid composition of claim 1, wherein the derivatives thereof are selected from the group consisting of ethyl esters, triglycerides, free fatty acids, and phospholipids of omega-3-fatty acids.

11. The omega-3-fatty acid composition of claim 1, further comprising: one or more antioxidants selected from the group consisting of α-tocopherol acetate, butylhydroxyanisole (BHA), and butylhydroxytoluene (BHT).

12. The omega-3-fatty acid composition of claim 1, wherein the composition spontaneously forms lyotropic liquid crystal structures in a gastrointestinal tract.

13. An omega-3-fatty acid oral preparation comprising the omega-3-fatty acid composition of any one of claims 1 to 12 and a pharmaceutically acceptable carrier.

14. An omega-3-fatty acid soft capsule comprising the omega-3-fatty acid composition of any one of claims 1 to 12 and a pharmaceutically acceptable carrier.

15. The soft capsule of claim 14, wherein the soft capsule is filled with 150 to 1500 mg of the omega-3-fatty acid composition.
